# EUROPEAN PATENT APPLICATION

(11) **EP 4 813 379 A1**
(43) Date of publication of application: **30.09.2026**
(21) Application number: 25166073.4
(22) Date of filing: 25.03.2025
(51) Int. Cl.: A61K 31/132, A61K 31/407, A61K 45/06, A61P 31/04, A61P 31/06

(54) **POLYAMINE DERIVATIVES FOR USE IN THE TREATMENT OF BACTERIAL INFECTIONS**

(71) Applicant: Universite De Fribourg, 1700 Fribourg (CH)
(72) Inventor: NORDMANN, Patrice, 1700 Fribourg (CH)
(74) Representative: ABREMA SA

(57) **Abstract**

The present invention concerns a compound for use in the treatment of bacterial infections and a method of treatment comprising administering the compound. In an embodiment, the compound is a polyamine for example trientine, and most preferably TETA-4HCL or TETA-2HCL. The compound is preferably used as an inhibitor of carbapenemases, in particular metallo-β-lactamases (MBLs). In a preferred embodiment, the compound is used in combination with an antibiotic, preferably a carbapenem antibiotic.

## Description

### Technical Field

The present invention relates to compounds for use as medicaments, and in particular in the treatment of bacterial infections and/or diseases caused by bacteria. More specifically, the invention relates to the treatment of infections and diseases caused by carbapenem-resistant bacteria. The invention further relates to methods of treatment, an *in vitro* method of detecting carbapenemase-producing bacteria and a kit for detecting the presence of such bacteria.

### Background Art and the Solution Approach of the Invention

Carbapenem-resistant Gram-negative bacteria have become a worldwide concern for public health¹. When resistant to carbapenems, important pathogens such as Enterobacterales, *P. aeruginosa,* and *A. baumannii* are responsible for increasing morbidity and mortality, longer hospitalization stay, and higher health costs¹⁻³, characteristics that categorize them to be included in the World Health Organization (WHO) top priority list of critical pathogens for research and development of new antibiotics⁴. Together, when resistant to carbapenems, these three pathogen species are responsible for more than 200,000 deaths worldwide in 2019⁵.

Carbapenemase expression is one of the most frequent mechanisms of resistance to carbapenems and is extensively reported in Enterobacterales, *P. aeruginosa,* and *A. baumannii.* Carbapenemases are β-lactamases capable of hydrolyzing almost all broad-spectrum β-lactams, including expanded-spectrum cephalosporins and carbapenems such as ertapenem, imipenem, and meropenem. These enzymes are mainly encoded on plasmids and are transmissible, contributing to the dissemination of acquired resistance⁶.

They can be classified by the Ambler classification system based on their catalytic domain and substrate preference⁷. The group B enzymes, namely metallo-β-lactamases (MBLs) are the only enzymes that have zinc in their active site, instead of the serine present in the active sites of the classes A, and D, namely serine β-lactamases (SBL)⁸. The most frequent and clinically relevant plasmid-encoded class A SBLs are the KPCs (***K**lebsiella **P**neumoniae* **C**arbapenemase)⁹. KPC enzymes are predominant in *Enterobacterales,* especially *K. pneumoniae* and are also identified in *P. aeruginosa⁹.* Its variant KPC-2 was first described in 2003 from the US and has been extensively reported worldwide in *K. pneumoniae*^{10,11}, mostly as a source of nosocomial infections.

The class D SBL, also known as oxacillinases with carbapenemase properties, are extensively reported in Enterobacterales and *A. baumannii*¹². The most prevalent representatives of this SBL class in Enterobacterales include the OXA-48 and related variants. OXA-48 was first described by my group, being recovered from a patient infected with K. *pneumoniae* in Turkey, in 2001¹³. These OXA-48-like enzymes are endemic in regions like Europe, the Indian subcontinent, and North Africa, and are a frequent cause of nosocomial infections^{14,15}. The oxacillinases with carbapenemase activity in *A. baumannii* are quite specific to that species and include OXA-23, OXA-40, and OXA-58 derivatives. OXA-23 is the most frequent enzyme associated with carbapenem resistance in *A. baumannii* and has been identified in outbreaks worldwide⁹. Unlike Enterobacterales and *P. aeruginosa,* carbapenem resistance in *A. baumannii* is always associated with the expression of carbapenemases.

MBLs are divided into three subclasses (B1, B2, and B3) based on differences in amino acid contents at several structural sites, such as active sites, loop structure, substrate affinity, zinc content, and ligands^{16,17}. The B1 subclass (e.g. NDM, VIM, IMP, SIM, DIM, GIM, SPM, KHM) comprises by far the most clinically significant clinically MBLs and largely plasmid-borne, a feature that allows their genes to be transferred between bacterial species¹⁶. The class B2 is represented by the narrow-spectrum chromosomal-borne CphA enzyme present in *Aeromonas* spp¹⁸. The B3 subclass group includes the intrinsic and chromosomal-borne carbapenemases reported in *Stenotrophomonas maltophilia* and *Elizabethkingia meningoseptica,* L1 and GOB¹⁹⁻²¹, respectively. This B3 class also includes some plasmid-borne representatives such as SMB (*Serratia* metallo-lactamase) and AIM (Adelaide imipenemase)^{22,23}. Those B2 and B3 subclass enzymes are less clinically significant than the plasmid mediated B1 members, except the intrinsic L1 enzyme from *S. maltophilia.*

The acquired MBLs (B1 class) were first described in Japan in 1991, recovered from a clinical isolate of *P. aeruginosa,* and named IMP-1 (imipenemase)²⁴. IMP-like enzymes can be found among Enterobacterales, *P. aeruginosa, and A. baumannii*^{9,24}. The VIM-type (Verona imipenemase) was first discovered in Italy²⁵. Most of the VIM-1-like are rarely identified Enterobacterales, while the VIM-2-type is often associated with *P. aeruginosa*²⁶*.* VIM-2 was also characterized by our group from a blood culture of a 39-year-old woman previously treated with imipenem in France, in 1996²⁶. It is now it is identified as the most prevalent carbapenemase in that species. That was the second report of carbapenem-hydrolyzing MBL characterized from *P. aeruginosa* outside Japan²⁶.

Lastly, the first case of NDM (New Delhi metallo-β-lactamase) was reported in 2009 in a Swedish patient of Indian origin who was hospitalized in Sweden after returning from New Delhi²⁷. It was recovered from the urine of that patient and identified as an NDM-1²⁷. The SPM-1 (São Paulo metallo-β-lactamase) is unique to South America and widely distributed in *P. aeruginosa* recovered in Brazil²⁸.

More than 30 years have passed since the first description of MBLs, have been now reported in a large variety of clinically significant Gram-negatives including Enterobacterales species, *P. aeruginosa, A. baumannii, S. maltophilia,* and *Achromobacter* spp.²⁹. The mortality rates for hospitalized patients with an infection caused by NDM-producing *Klebsiella pneumoniae,* NDM-producing *A. baumannii,* IMP-producing Enterobacterales, VIM-producing *P*. *aeruginosa,* and SPM-producing *P. aeruginosa,* can reach 42%, 42%, 39%, 48%, and 51%, respectively³⁰⁻³⁴. Table 1 summarizes the presence of MBLs in Enterobacterales, *P*. *aeruginosa, and A. baumannii.*

**Table 1. Acquired metallo-β-lactamase detected in Enterobacterales, P. aeruginosa, and A. baumannii**

| Organism | NDM | VIM | IMP | Other Sporadic MBLs |
|---|---|---|---|---|
| *Enterobacterales* | | | | GIM, SIM, SPM, KHM |
| *P. aeruginosa* | | | | SPM, GIM, PFM, DIM |
| *A. baumannii* | | | | SIM-1 |

In Table 1, worldwide prevalence is shown according to the intensity of the grey color.

Dark grey indicates higher prevalence, medium grey indicates medium prevalence, light grey indicates lower prevalence. NDM: New Delhi metallo-β-lactamase, VIM; Verona imipenemase, IMP; Imipenemase, GIM; German imipenemase, SIM: Seoul imipenemase, SPM: São Paulo metallo-β-lactamase: KHM, Kyorin Health Science MBL 1, PFM: *Pseudomonas fluorescens* metallo-β-lactamase DIM, Dutch imipenemase
MBLs are of particular concern because they can hydrolyze virtually all the β-lactams except the monobactam aztreonam. They are ubiquitous and can be recovered from both nosocomial and environmental sources. Additionally, they are highly transferable, they have a fast pace of developing new variants. Moreover, there is a lack of clinically useful MBL inhibitors^{9,35}. Unlike SBLs, currently, there is not a single clinical inhibitor of MBLs available for treating infections caused by these pathogens. While a vast majority of newly developed β-lactamase inhibitors have action against classes A, C, and D, they are not effective against MBL enzymes because of being resistant to the B-lactam and resistant to the inhibitor of the combination. For instance, Ceftazidime-Avibactam, Meropenem-Vaboractam, and Imipenem-Relebactam are active against classes A, C while Ceftazidime-Avibactam and Imipenem-Relebactam are active against some class D carbapenemases (OXA-48 variants)³⁶⁻³⁸. Aztreonam-Avibactam (AZA) is active against most MBL producers because MBLs do not hydrolyze aztreonam and avibactam inhibits the activity of ESBLs and AmpC. It represents, so far, one of the best potential treatments for infections due to MBL producers. However, AZA is inactive against *A. baumannii in vitro* because aztreonam has no target in that species and less reliability active against MBL-producing *P. aeruginosa,* because aztreonam has weak antipseudomonas activity³⁹. Resistance to AZA in Enterobacterales has been reported in some instances mostly associated with changes in the target of aztreonam, penicillin-binding protein 3⁴⁰.

Inhibitors of MBLs that are in development include Taniborbactam and Xeruborbactam. Cefepime-Taniborbactam is in phase 3 clinical trials⁴². However, Cefepime-Taniborbacm has no activity against IMP-like MBLs^{39,42}. The MBL inhibitor Xeruborbactam is in clinical trial phase 1, but *in vitro* tests in combination with its most likely partner, meropenem, have presented promising results, being considered the β-lactam/β-lactamase inhibitor with the broadest spectrum of inhibition. It is active against many MBLs, in order of highest to lowest activity, NDM, VIM, and IMP, contrary to Cefepime-Taniborbactam that has no activity against IMP, as mentioned above⁴³. A part of the lack of activity against IMPs, cefepime-taniborbactam resistance has been attributed to the presence of the NDM-9 variant in Enterobacterales and *A. baumannii,* and its dissemination was reported in clinical strains from Italy, Switzerland, and the USA, and environmental source (water) in South Korea⁴⁴. Very recently, I found (unpublished data) that a series of IMP enzymes are actually resistant also to the inhibition by xeruborbactam and that IMP-producing *P. aeruginosa* strains are resistant to meropenem-xeruborbactam due to efflux of xeruborbactam by naturally-expressed efflux pump MexAB-OprM.

Recently, I have identified a novel inhibitor of MBL, dimercaptosuccinic acid (DMSA), which is a zinc chelator^{45,46} This molecule has been used for decades to treat lead poisoning. There is no active company currently promoting actively the use of that molecule. EP2979694B1 discloses the potential use of DMSA in MBL inhibition. Proof-of-concept of its efficacy has been established in mouse models of peritonitis with Enterobacterales producing MBLs⁴⁵, and very recently with *P. aeruginosa* expressing NDM-1 or VIM-2⁴⁷.

Other zinc chelators are being evaluated (mostly *in-vitro* studies) for inhibiting some of these MBLs^{48,49}. They belong to different families of chemical and synthetic molecules such as tetraacetic acid, tretraazacyclododecane, ethylenediamine, dipicolonic acid and the natural inhibitor of MBLs, aspergillomarasmine extracted from *Aspergillus versicolor*⁵⁰*.* This natural product is an aminopolycarboxylic acid that functions as an effective inhibitor of clinically significant MBLs. However, it is less effective against some clinically significant MBLs such NDM-6 and IMP-7, which are widespread worldwide.

From the above, it becomes apparent that there is no universal molecule that can inhibit all carbapenemases and/or all metallo-β-lactamases (MBLs). There is a need for further compounds or compositions that are useful in the treatment of bacterials, including infections of carbapenem-resistant, Gram-negative bacteria.

There is a need for new treatments against infections of Enterobacterales, *Pseudomonas aeruginosa* (*P. aeruginosa*) and *Acinetobacter baumanii (A. baumanii), Stenotrophomonas maltophilia (S. maltophilia),* and *Achromobacter* spp..

There is in particular a need for new inhibitors of β-lactamases such as carbapenemases, especially inhibitors that are suitable for medical uses. As mentioned above, there is a lack of clinically useful MBL inhibitors, such as NDM, IMP and VIM inhibitors. There is a need for inhibitors that can be used in combination with β-lactam antibiotics, such as carabapenems, for treating bacterial infections.

There is also a need for new diagnostic tests and methods that enable the rapid detection of carbapenem-producing bacteria. Ideally, the type or subtype of carbapenemase produced by a bacterium can be detected.

The present invention addresses the problems depicted above.

### Summary of the Invention

The present inventor found that certain polyamine compounds are useful as inhibitors of β-lactamases, in particular of carbapenemases, for example of metallo-β-lactamases (MBLs).

In an aspect, the invention provides a compound comprising a moiety of formula (I) below, and/or of a pharmaceutically acceptable salt thereof: 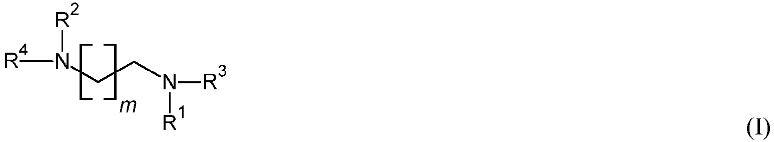 wherein
m is 0 and integers of 1-5, preferably 1;
R¹, R², R³, and R⁴ are independently selected from H and substituents of formulae (1) below 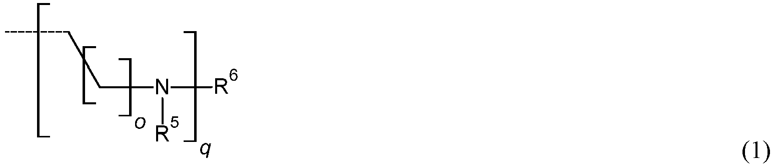
wherein
o is selected from 0 and integers of 1-5, preferably 1;
q is an integer of 1-5;
R⁵ and R⁶ are independently selected from H and substituents of formula (2) below 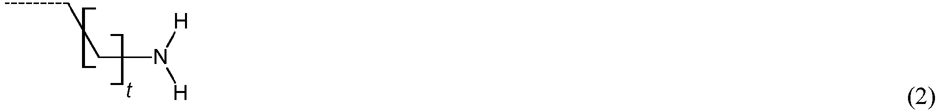
wherein
t is selected from 0 and integers of 1-5.

In an aspect, the invention provides a polyamine compound.

In an aspect, the invention provides the compounds of the invention for use as a medicament.

In an aspect, the invention provides the compounds of the invention for use in the treatment of a bacterial infection and/or a disease caused by bacteria.

In an aspect, the invention provides the compounds of the invention for use as an inhibitors of carbapenemases, in particular MBLs, preferably subclass B1 MBLs. The compounds may be used *in vivo* or *in vitro* as such inhibitors.

In an aspect, the invention provides the compounds of the invention for use in the treatment of infections of and/or diseases caused by carbapenemase-producing bacteria and/or carbapenem-resistant bacteria.

In an aspect, the invention provides the compounds of the invention for use in the treatment of infections of and/or diseases caused by bacteria producing one or more selected from class B, A, C and D carbapenemases, including combinations of two or more of the aforementioned.

In an aspect, the invention provides the compounds of the invention for use in the treatment of infections of and/or diseases caused by metallo-β-lactamase (MBL) producing bacteria.

In an aspect, the invention provides the compounds of the invention for use in the treatment of infections of and/or diseases caused by bacteria producing an MBL selected from subclass B1, B2 or B3, or a combination of two or more of these subclasses, or any one or more of these subclasses in combination with a carbapenemase of any other class (A, C, D).

In an aspect, the invention provides the compounds of the invention for use in the treatment of infections of and/or diseases caused by bacteria producing an MBL subclass B1 carbapenemase.

In an aspect, the invention provides method for treating a bacterial infection, the method comprising administering, to a subject in need thereof, a therapeutically effective amount of the compound of the invention.

In an aspect, the invention provides an *in vitro* and/or *ex vivo* test kit for detecting the presence of a carbapenemase, preferably a metallo-β-lactamase (MBL), the kit comprising the compound of the invention.

In an aspect, the invention provides an *in vitro* and/or *ex vivo* method for detecting the presence of a metallo-β-lactamase (MBL), the method comprising:
- providing a sample comprising a test sample and the compound of the invention;
- detecting the presence of said MBL if an activity of said MBL is inhibited due to the presence of said compound of the invention.

Further aspects and preferred embodiments of the invention are defined herein below and in the appended claims. Further features and advantages of the invention will become apparent to the skilled person from the description of the preferred embodiments given below.

### Brief Description of the Drawings

**Figure 1** is a graph showing growth curves of *E. coli* MG1655 expressing the NDM-5 carbapenemase with meropenem (MEM) alone, TETA-4HCL (T4H2S) alone or combinations of MEM with TETA-4HCL at various concentrations.
**Figure 2** is a photograph showing the outcome of a diagnostic test in accordance with an embodiment of the invention.
**Figure 3** is a photograph showing specific inhibition of MBL carbapenemase by TETA 4HCl, in accordance with an embodiment of the invention.

### Detailed Description of the Preferred Embodiments

For the purpose of the present specification, the expression "comprising" and its various grammatical forms is intended to mean "includes, amongst other". It is not intended to mean "consisting only of".

In a preferred embodiment, the compound of the invention is a polyamine and/or a compound comprising a moiety of formula (I). The moiety of formula (I) is preferably a polyamine. In the present specification, reference to the "compound" is generally understood as a reference to the "compound of the invention".

In some embodiments, the invention relates to compounds comprising a moiety of formula (I), protonated and deprotonated forms thereof, and/or of a pharmaceutically acceptable salt thereof. 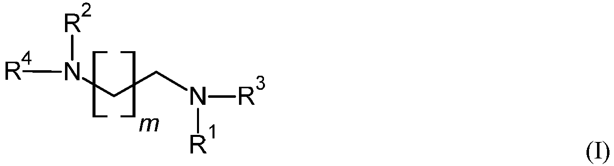 wherein
m is selected from 0 and integers of 1-5.

In a preferred embodiment, m is an integer selected from 1, 2 and 3. More preferably, m is 1 or 2, and most preferably m = 1.

R¹, R², R³, and R⁴ are independently selected from H and substituents of formula (1) below 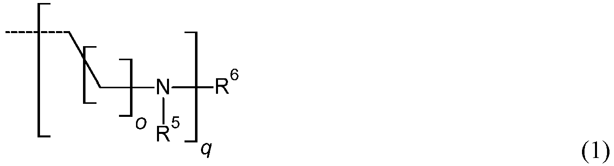 wherein
o is selected from 0 and integers of 1-5, preferably 1;
q is an integer of 1-5;
R⁵ and R⁶ are independently selected from H and substituents of formula (2) below 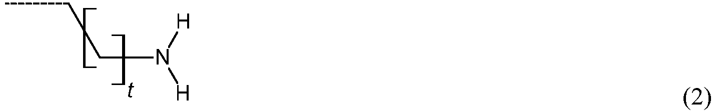
wherein
t is selected from 0 and integers of 1-5.

The dotted lines in the structures disclosed herein represent the single bond that bridges the atoms of the moiety comprising the substituent and the corresponding substituent, respectively. For example, the dotted line in formula (1) represents the single bond linking a nitrogen atom in the moiety of formula (I) to the carbon atom in formula (1), by which the substituent is linked to the nitrogen atom. Therefore, in the substituent of formula (1), if o and q are 1 and R⁵ and R⁶ are both H, the substituent has two carbons.

In a preferred embodiment, in the substituent of formula (2), t is 1, 2 or 3, and most preferably t is 1.

In a preferred embodiment, in the substituent of formula (1), o is 1, 2 or 3, more preferably 1 or 2, and most preferably it is 1.

In a preferred embodiment, in the substituent of formula (1), q is 1, 2 or 3, more preferably 1 or 2, and most preferably it is 1.

Preferably, R⁵ and R⁶ of the substituent of formula (1) is H, q is 1, 2 or 3, more preferably 1 or 2, and most preferably it is 1.

In a preferred embodiment, R⁵ and R⁶, is as far as present, are H.

In a preferred embodiment, R¹, R², R³, and R⁴, in the moiety of formula (I), are independently selected from H and substituents of formulae (3), (4), and (5) below:

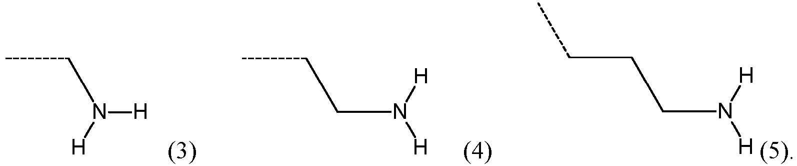

In a preferred embodiment, R¹, R², R³, and R⁴, are independently selected from H and substituents of formula (4).

In a preferred embodiment, R¹ and R² are both H.

In a preferred embodiment, R³ and R⁴ are independently selected of substituents of formula (2), and more preferably from substituents of formula (3)-(5) above.

In a preferred embodiment, R¹ and R² are H, and R³, and R⁴ are independently selected from substituents of formulae (3), (4), and (5).

In a preferred embodiment, m is 1, 2 or 3; R¹ and R² are both H; R³ is selected from H and from substituents of formulae (3)-(5); and, R⁴ is selected from substituents of formulae (3)-(5).

In yet a preferred embodiment, m is 1 or 2, R¹ and R² are both H; R³ is selected from H and from substituents of formulae (4) and (5).

In a preferred embodiment, the moiety of formula (I) is or comprises one or more polyamines, preferably one or more ethylene amines, and most preferably triethylenetetramine (TETA).

In a preferred embodiment, the moiety of formula (I) is or comprises one or more selected from triethylenetetramine (TETA), also know as trientine, spermine and spermidine.

In an embodiment, the moiety of formula (I) is or comprises one or more selected from the group of compounds (V), (VI) and (VII) below.

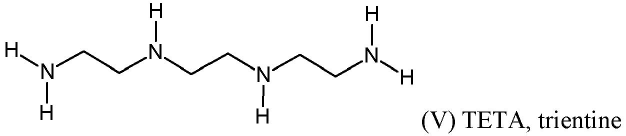

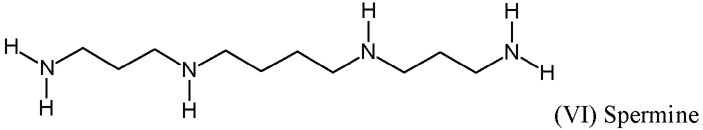

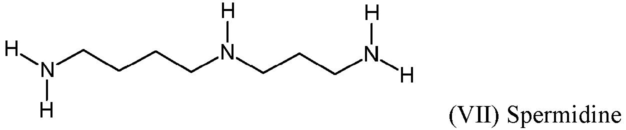

In some embodiments, the compound of the invention comprises a polyamine. The polyamine preferably comprises a linear, branched and/or circular chain of nitrogen and carbon atoms, in which said nitrogen atoms form primary or secondary amine groups. In an embodiment, the polyamine comprises 3-25 carbons and 2-15 nitrogen atoms, preferably 4-17 carbons and 2-10 nitrogen atoms, and most preferably 5-12 carbons and 3-7 nitrogen atoms.

Preferably, the polyamine is a linear compound.

Preferably, the polyamine is a saturated compound.

Preferably, the terminal atoms of said linear or branched chain are nitrogen atoms forming primary amine groups.

In an embodiment, the compound of the invention is a polyethylenepolyamine. Preferably, the compound of the invention may comprise 2- 6 amine groups and 2-6 ethylene groups. The compound of the invention may be a triethylenetetramine or a tetramethylenepentamine. Generally, the compound of the invention may be of the formula x-ethylene-y-amine, where x and y represent the numbers of ethylene and amine moieties, respectively. Preferably, y = x+1. In an embodiment, x is 2, 3, 4 or 5, preferably 3, and y is 3, 4, 5 or 6, preferably 4.

Preferably, one, several or all of the terminal groups of the compound of the invention are primary amine groups.

Preferably, the polyamine is the moiety of formula (I).

In a preferred embodiment, the compound of the invention is provided in the form of an amine salt.

Preferably, the amine salt is the salt of the polyamine and/or the moiety of formula (I) with an organic or inorganic acid.

Preferably, the amine salt is the salt of the polyamine and/or of the moiety of formula (I) with an acid having a pKa value of < 5, preferably <4, more preferably < 3 and most preferably <2.

Preferably, the amine salt is the salt of the polyamine and/or of the moiety of formula (I) with a strong acid and/or with an acid having a pKa value of < 0.

Preferably, the amine salt is the salt of the polyamine and/or of the moiety of formula (I) with a pharmaceutically acceptable acid.

In a preferred embodiment, the compound comprises a hydrochloride salt of the moiety of formula (I) and/or of the polyamine.

In a most preferred embodiment, the compound of the invention is selected from TETA-4HCl (TETA tretrahydrochloride) and TETA-2HCl (TETA dihydrochloride). TETA-4HCl is most preferred.

The compounds of the invention are useful in the treatment of bacterial infections and/or diseases caused by bacteria.

In some embodiments, the bacterial infection and/or the disease caused by a bacterium, includes one or more of the following: Tuberculosis, Pneumonia, Strep Throat, Whooping Cough, Cellulitis, Impetigo, Leprosy, Hansen's Disease, Salmonellosis, Cholera, Vomitus, Diarrhea, Botulism, Syphilis, Urinary Tract Infections, meningitis, Tetanus, and Septicemia.

For the purpose of the present invention, bacterial infections include Respiratory Infections (pneumonia etc), Skin and Soft Tissue Infections (cellulitis, etc), Gastrointestinal and Foodborne Infections, Sexually Transmitted Infections, Urinary Tract Infections, Nervous System Infections, and Bloodborne Infections.

In an embodiment, the bacterial infection is an infection caused by one or more selected from Enterobacterales, *Pseudomonas aeruginosa* (*P. aeruginosa*) and *Acinetobacter baumanii* (*A. baumanii*), *Stenotrophomonas maltophilia* (*S. maltophilia*), and *Achromobacter* spp. Infections caused by other bacteria are not excluded.

The compounds of the invention are in particular inhibitors of at least certain β-lactamases, in particular carbapenemases, more specifically MBLs. MBLs include NDM, VIM, IMP, GIM, SIM, SPM, KHM, PFM, DIM and SIM-1, for example, as specified in **Table 1** above.

In a preferred embodiment, the compounds of the invention are inhibitors of the B1 subclass of MBLs.

In an embodiment, the compounds of the invention are inhibitors of the MBLs NDM, VIM, IMP, SIM, SIM-1, DIM, GIM, SPM, and KHM.

In a preferred embodiment, the compounds of the invention are in particular inhibitors of NDM, VIM, IMP, and most preferably of NDM MBLs.

In accordance with an embodiment, the compounds of the invention are used as inhibitors of the above β-lactamases.

In accordance with the above, the compounds of the invention are particularly useful for treating of infections of carabapenemase producing bacteria and/or carbapenem-resistant bacteria, and/or for treating diseases caused by such bacteria.

In an embodiment, the compounds of the invention are used for treating infections of bacteria producing class A, B, C and/or D carbapenemases, or combinations comprising two or more different carbapenemases of the same or different classes.

In a preferred embodiment, the compounds of the invention are used for treating infections of metallo-β-lactamase (MBL) producing bacteria, and/or for treating diseases caused by such bacteria.

In an embodiment, the compounds of the invention are zinc chelators and/or have zinc chelating properties *in vitro* and/or *in vivo.* Preferably, the compounds of the invention have zinc chelating properties that are superior or at least equal to those of DMSA.

In an embodiment, the compounds of the invention are used for treating infections of bacteria producing any one or several selected from subclass B1, B2, and/or B3 metallo-β-lactamase (MBL), and for treating diseases caused by such bacteria.

Subclass B2, for example, is represented by the narrow-spectrum chromosomal-borne CphA enzyme present in *Aeromonas* spp. The B3 subclass group includes the intrinsic and chromosomal-borne carbapenemases reported in *Stenotrophomonas maltophilia* and *Elizabethkingia meningoseptica,* L1 and GOB, and also plasmid-borne representatives such as SMB (*Serratia* metallo-lactamase) and AIM (Adelaide imipenemase).

In an embodiment, the compounds of the invention are used for treating infections of MBL subclass B1 producing bacteria, and/or for treating diseases caused by such bacteria.

In an embodiment, the compounds of the invention are used for treating infections of bacteria producing one or more selected from NDM, VIM, IMP, SIM, DIM, GIM, SPM, KHM, and/or for treating diseases caused by such bacteria. The compounds of the invention may be used for infections of bacteria producing one or more of the aforementioned, optionally in combination with other carbapenemases, of class B or of any one or several carbapenemases selected independently from class A, C, and D.

In an embodiment, the compounds of the invention are used for treating infections of bacteria producing one or more selected from the group consisting of: New Delhi Metallo-β-lactamase (NDM), imipenemase (IMP) and Verona imipenemase (VIM), and/or for treating diseases caused by such bacteria.

In an embodiment, the invention provides a combination treatment. Accordingly, the compound of the invention is preferably administered in combination with an antibiotic, preferably a β-lactam antibiotic, and most preferably a carbapenem antibiotic.

In an embodiment, the antibiotic is selected from the group consisting of penicillin compounds, cephalosporines, carbapenems, and monobactams. Exemplary penicillin compounds include amino penicillins and are disclosed in WO2017/060427, in particular starting on page 13, lines 1-18. Exemplary cephalosporins and carbapenems are disclosed on the same page, lines 26-28. These disclosures are expressly incorporated herein by references.

In a preferred embodiment, the antibiotic is selected from meropenem, ceftazidime, and imipenem.

The antibiotic may be administered simultaneously with the compound of the invention. For example, the compound of the invention and the antibiotic may be contained in the same pharmaceutical composition. In an embodiment enabling simultaneous administration, for example, the compound of the invention and the antibiotic are contained in the same tablet, pill or capsule.

In another embodiment, the antibiotic and the compound of the invention are administered separately, independently, and/or consecutively.

For example, the invention encompasses the administration of two pharmaceutical compositions, a first composition comprising the compound of the invention and the second composition comprising the compound of the invention. The invention encompasses that the two compositions are administered simultaneously. In other embodiments, administration of the two compositions is takes place in a timely separate and/or spaced apart manner. In an embodiment, the two compositions are administered over the same or overlapping time periods, but otherwise independently one from the other, based on a treatment scheme, which is established independently for the compound of the invention and the antibiotic. For example, the antibiotic may be administered in one or two daily doses, whereas the compound of the invention may be administered in 2, 3, 4, 5, 6, 7, 8 or even more daily doses.

Furthermore, the administration form of the first and second compositions may be the same or different. For example, the antibiotic may be administered orally, rectally, or parentally, for example intravenously. The administration form of the antibiotic will strongly depend on the particular antibiotic and may be chosen independently in accordance with the antibiotic's preferred form of administration.

The compound of the invention is preferably provided and/or contained in the form of a pharmaceutical composition. The pharmaceutical composition comprises the compound of the invention and at least one pharmaceutically acceptable excipient or carrier. The excipient will be selected in accordance with the form of administration. The composition may comprise a solid excipient, suitable for forming a tablet, capsule or pill, or may be liquid and may comprise, for example, water or other suitable solvents, in case of administration in the form of a liquid, for example in case of parenteral such as intravenous administration.

Exemplary excipients for tablet formulations comprise diluents or fillers, binders, disintegrants, lubricants, glidants, coating agents, colorants and flavouring agents. The pharmaceutical composition may comprise one or more of the aforementioned and preferably comprises at least one. Typical fillers include one or more of microcrystalline cellulose, lactose dicalcium phosphate, starch and mannitol. Typical binders are polyvinylpyrrolidone (PVP), hydroxypropyl methylcellulose (HPMC), gelatin, and starch paste. Typical disintegrants include sodium starch glycolate, croscarmellose sodium, crospovidone, and microcrystalline cellulose. Lubricants may be selected from magnesium stearate, stearic acid, and talc, for example. Typical coating agents may be selected from HPMC, polyethylene glycol (PEG), titanium dioxide and shellac.

The compound of the invention and/or the pharmaceutical compositions comprising the compound may be administered in any suitable form of administration, selected, for example, from one or more of the group of oral, nasal, buccal, sublingual, rectal, vaginal, parenteral, intravenous, intramuscular, subcutaneous, intraperitoneal, intradermal, topical, and transdermal administration. Preferably, the compound of the invention is administered in a systemic manner, allowing the compound to enter the bloodstream and circulate throughout the body, affecting multiple organs or tissues.

In an embodiment, the compound of the invention and/or the pharmaceutical compositions is administered at least once per day. In some embodiments, the daily administration dose is distributed over the day, and may include, several administrations per day. In an embodiment, the compound of the invention is administered in 1, 2, 3, 4, 5, 6, 7, 8 or even more daily doses.

In an embodiment, the compound of the invention is administered every 2-24 hours, for example two hours (for example in 12 daily doses), every three hours, every four hours, every 5 hours, every 6 hours, every 8 hours (3 daily doses) or every 12 hours (twice a day).

In an embodiment, the compound of the invention is administered at a therapeutically effective dose.

The polyamine of formula (I), for example the trientine (not including hydrochlorides or other salts or ions, in particular anions), may be administered at a dose of 1-35 g/day, preferably 1.5-30 g/day, more preferably 2-25 g/day, even most preferably 2.5-20 g/day, and most preferably 3-15 g/day.

The dose may be adapted to the particular situation, taking into account several factors, including, for example, the body weight of the person to be treated. It may also be considered whether the subject is female or male.

In mg per kg bodyweight of the subject, the daily dose of the polyamine of formula (I), for example the trientine (not including hydrochlorides or other salts or ions, in particular anions), may be 15-550 mg/kg/day, preferably 30-350 mg/kg/day, more preferably 50-300 mg/kg/day, even more preferably 70-250 mg/kg day, and most preferably 80-200 mg/kg/day.

The above values may apply, for example, to a human adult, and preferably for a treatment duration as indicated above, preferably 3 weeks (21 days) or less. For human non-adults, the upper limit is preferably lower and/or doses may be applied that are closer to the lower limits of the indicated ranges.

In case the polyamine is administered in the form of TETA 4HCL, the above amounts may be doubled, as trientine provides 50% of the weight of TETA 4HCL.

In an embodiment, TETA 4HCL is administered at a daily dose of 0.2 g to 50g, preferably 3 g to 40 g, more preferably 4 g to 30 g, even more preferably 5 to 20g, and most preferably 6 to 15 g.

In a preferred embodiment, TETA 4HCL is administered at a dose of 5 to 30 g per day.

In mg per kg bodyweight of the subject, the daily dose of TETA 4HCL is preferably 30-700mg/kg/day, preferably 70-600mg/kg/day, more preferably 100-550 mg/kg/day, even more preferably 140-500mg/kg/day, and most preferably 150-450 mg/kg/day.

In case the polyamine is administered in the form of TETA 2HCL, the trientine base provides about 2/3 of the weight, such that the amounts indicated for the polyamine alone (e.g. trientine alone, not including the hydrochlorides) are preferably multiplied by 3/2.

In an embodiment, TETA 2HCL is administered at a daily dose of about 1.5 g to 45g, preferably about 1.2.5 g to 40 g, more preferably 3 g to 35 g, even more preferably 4g to 30g, and most preferably 5.0 to 25 g.

In a preferred embodiment, TETA 2HCL is administered at a dose of 4 to 25 g per day.

In mg per kg bodyweight of the subject, the daily dose of TETA 2HCL is preferably 25-650mg/kg/day, preferably 50-550mg/kg/day, more preferably 80-450 mg/kg/day, even more preferably 100-400mg/kg/day, and most preferably 120-350 mg/kg/day.

Preferably, the administration dose is adapted to take into account the severity of the infection, prognosis, and the side effects associated with the administration of the polyamine at higher doses, taking also into account the treatment duration. For example, side effects caused by the polyamine compound may be found acceptable in the case of very severe infections and absence of alternative treatments, such that doses at the higher end of the ranges indicated above may be administered. Similarly, doses at the upper end of the ranges may also be administered in case of shorter treatment duration, for example 15 days or less.

In an embodiment, TETA 4CHl is administered at a dose that is suitable to achieve a serum concentration of 20-100 µg/ml, preferably 25-75 µg/ml.

In an embodiment, TETA 2CHl is administered at a dose that is suitable to achieve a serum concentration of 15-75 µg/ml, preferably 15-65 µg/ml.

In another embodiment, these serum concentrations are preferably achieved for a period of 1 hour or longer, preferably 1-3 hours or more, more preferably 2-6 hours or more, and most preferably 4-12 hours or more.

The compound of the invention is preferably administered rapidly or immediately upon detection of the bacterial invention. In some embodiments, the compound of the invention is administered after detecting the presence of MBL, and in particular a subclass B1 MBL, for example as specified herein above, is confirmed and/or detected in the patient to be treated.

In this regard, the invention may include detecting the presence of carbapenemase and/or determining the class and/or even the subtype of the carbapenemase expressed by the bacterium at the origin of the infection. For example, the invention encompasses administering the compound of the invention only in case an MBL is detected, for example in cases where subtype B1 MBL is detected, for example in case a an NDM, IMP or VIM is detected.

The compound of the invention is preferably administered for a defined time period, for example during a period of at least 4 days and up to 4 weeks or less, preferably at least 5 days and up to 21 days or less, and more preferably at least 6 days and up to 15 days or less.

The invention also encompasses a method of treatment. The method comprises administering to a subject in need thereof, an effective amount of the compound of the invention. For example, the effective amount is pharmaceutically effective, resulting in effective treatment of the bacterial infection and/or the disease.

The subject in need is preferably a human or animal subject suffering from a disease and/or infections as specified elsewhere in this specification. Exemplary pharmaceutically effective amounts have been specified elsewhere, for example for TETA-4HCL. It is noted that the dose generally also depends on the subject, age, and may depend on the gender and/or other factors, and will be selected by the person skilled in the art to achieve the desired therapeutic outcome.

Exemplary pharmaceutical compositions comprise at least the compound of the invention and a pharmaceutically acceptable carrier and/or excipient. Excipients have been disclosed elsewhere in this specification.

In some embodiments, the invention encompasses a test kit comprising the compound of the present invention. In a preferred embodiment, the test kit allows detecting the presence of an MBL, in particular of a subclass B1 MBL, for example as further specified elsewhere in this specification. Preferably, the test kit allows at least detecting the presence of a β-lactamase and preferably allows to distinguish between carbapenemases and other β-lactamases. In a further preferred embodiment, the test kit allows distinguishing between different classes of carbapenemases, such as, for example, between class A, class B, and class D.

Preferably, the test kit is an *in vitro* and/or *ex vivo* test kit. In an embodiment, the test kit comprises the compound of the invention. In some embodiments, the test kit further comprises a solution, for example a buffer solution, such as a lysis buffer solution.

In some embodiments, the test kit comprises at least one substrate for the carbapenemase, in particular the MBL, to be detected.

In some embodiments, the test kit comprises a marker ingredient, which is suitable to provide a readable signal allowing a reading of the test outcome. In a preferred embodiment, the marker is a color marker, enabling the reading of the test outcome by visual inspection and/or by the generation of an optical signal, for example a change of color. In an embodiment, the marker is preferably chromogenic.

In a preferred embodiment, the marker and the substrate are the same compound, for example the substrate is chromogenic. For example, the substrate and/or marker is nitrocefin.

In some embodiments, the test kit comprises one or more inhibitors of carbapenemases, preferably at least one but preferably at least two, three, for or more different inhibitors. In an embodiment, the test kit comprises at least ertapenem, for example for detecting the presence of a carbapenemase (in absence of inhibition) or of another β-lactamase (in case there is inhibition of the substrate cleavage in the corresponding sample).

In a preferred embodiment, the test kit preferably comprises further, different inhibitors suitable to distinguish at least between classes A, B and D. For example, the test kit may comprise AVI (avibactam), which inhibits class A and class D carbapenemases, and VAB, which inhibits class D but not class A carbapenemases.

The test kit is preferably adapted and/or comprises equipment for receiving, treating, and/or analyzing samples. The sample preferably comprises bacteria that are subjected to the test, for example bacteria taken from a culture and/or pretreated and/or lysed bacteria. In some embodiments, the bacteria are directly taken from a sample taken from an individual, for example comprising a body liquid, such as blood, serum, utile, saliva.

In an embodiment, the test kit comprises a plurality of recipients, such as tubes, for receiving samples and/or kit reactants and/or buffers as detailed above. In an embodiment, the kit comprises at least a tube for receiving a sample and the MBL inhibitor in accordance with the invention, preferably TETA-4HCL.

In a preferred embodiment, the test kit is designed for detecting if a carbapenemase is present. For example, the test kit may comprise one, two or more additional test tubes, suitable for determining that a β-lactamase and/or a carbapenemase is present.

In a preferred embodiment, the test kit is designed for detecting a particular class or subclass of carbapenemase by the inhibition and thus absence or reduction of a detectable reaction.

In the test kit shown in examples, this is achieved by using the chromogenic substrate nitrocefin, which is hydrolyzed by all β-lactamases, and ertapenem (ETP), which inhibits all β-lactamases except carbapenems. A recipient 1 may be provided for receiving the chromogenic substrate without ETP and a recipient 2 comprising the substrate plus ETP. If substrate cleavage is detected in recipients 1 and 2, a carbapenemase is present. If the substrate cleavage is detected in recipient 1 only, there is a β-lactamase, but it is not a carbapenemase, as the β-lactamase is inhibited in recipient 2 only. Additional recipients and inhibitors may be used for distinguishing between different classes of carbapenems.

Preferably, at least one additional recipient (recipient 3) is provided for receiving the chromogenic substrate in addition to the MBL inhibitor of the present invention. If presence of carbapenemase is confirmed by recipients 1 and 2, absence of carbapenemase activity in recipient 3 (which may also contain ETP) indicates the presence of MBL.

The invention encompasses that the presence of carbapenemase may be detected otherwise than based on the nitrocefin/ETP system depicted above.

In a preferred embodiment, at least a recipient 4 is present, as well as a class A carbapenem inhibitor, such as vaborbactam and/or avibactam, which may be preloaded in the recipient or added when conducting the test.

In a still further preferred embodiment, a recipient 5 is present, as well as a class A and class D inhibitor, such as avibactam, which may be preloaded in the recipient or added when conducting the test.

In the various embodiments, some reactants, such as lysis buffer and one or more inhibitors, may be preloaded and/or already present in the recipients of the test kit. In some embodiments, one or some reactants are provided separately and need to be added to the appropriate recipient by a user, patient and/or medically trained person when using the test kit. In a preferred embodiment, at least the substrate is added when using the test kit, in particular after adding the sample of bacteria to each test tube.

In some embodiments, the diagnostic test further comprises a source of zing, such as ZnSO⁴, in particular for enabling reactions requiring zinc.

More generally, substrate consumption may be detected in other ways than by using a chromogenic substrate. In some embodiments, a color change may be induced by a change of the pH of the sample, which change of pH may be the result of the carbapenemase and/or β-lactamase activity. Other ways of translating the carbapenemase and/or β-lactamase activity into a detectable signal, which may be color or other, are encompassed by the invention.

### Examples

### Example 1: MIC values for TETA-4HCL alone and in combination with

First, we have studied the concentrations of TETA-4HCL that might increase sensitivity to the carbapenem antibiotic meropenem (MEM).

Minimal inhibitory concentrations (MIC) of TETA-4HCL alone was determined in Mueller-Hinton broth alone against *E. coli* MG1655 reference strains lacking carbapenemases according to the EUCAST guidelines³². This MIC value was 5000 µg/ml, much higher than the peak human serum concentration of 2-3 µg/ml TETA-4HCL observed after a regular treatment with TETA-4HCL for treating Wilson disease^{28,29}.

Then, MICs of MEM were determined for isogenic *E. coli* MG1655 strains expressing different carbapenemases (both MBL and non-MBL carbapenemases). The corresponding genes were cloned onto a same recombinant plasmid pUCP24 and expressed in *E. coli.*

MICs of MEM alone and with TETA-4HCL in checkerboard assays at different concentrations were determined by broth microdilution, according to EUCAST guidelines^{32,33}. The fractional inhibitory concentration index (FIC) was calculated and interpreted as follows:
Calculation of the FIC value: The MIC of drug A in combination / the MIC of drug A alone + the MIC of drug B in combination / the MIC drug B alone.

Interpretation of the FIC value:
FIC ≤ 0.5 = synergy; 0.5 < FIC ≤ 1 = additive; 1< FIC< 2 = indifference, and FIC≥ 2 = antagonis⁴.

The results are shown in **Table 2,** at the end of these Examples

As the table shows, **the addition of TETA-4HCL decreased the MIC values of MEM values at a concentration as low as 3 µg/ml for certain MBLs producers** (Table 2). This effect was evidenced for MBLs but not for non-MBL carbapenemase producers (OXA-18, KPC-3). Interestingly in some cases, full susceptibility to MEM was recovered after addition of TETA-4HCL even at those low-level concentrations (the susceptibility breakpoint for MEM in enterobacterales is 2 µg/ml). See for example the MIC value of 2 with MG1655/NDM-5 at MEM+TETA 4HCL, 3 ug/ml.

The efficacy of addition of TETA-4 HCL depended of the type of MBL (**Table 2**). Interestingly, this inhibitor was the most efficient against NDM producers, see the bottom three strains, which produce NDM-1, NDM-5 and NDM-9. NDM producers represent the most urgent threat among MBL producers.

One of them, NDM-5, is of special interest since (i) it possesses an highest carbapenemase activity among NDM-type enzymes, (ii) it is spreading rapidly among Enterobacterales in Europe, and (iii) and is often associated to resistance to the novel combination aztreonam-avibactam that is about to be marketed in Europe^{34,35}. This resistance trait is associated with structural modification of the penicillin binding protein 3 that is the target of aztreonam.

Generally, synergistic interaction (FIC ≤ 0.5) of TETA-4 HCL and MEM was regularly found with NDM producers, but also with VIP and IMP producers (see FIC values of *E. coli* clones 5-10 from the top in Table 2).

### Example 2: Time-kill assays against an NDM-1 producer

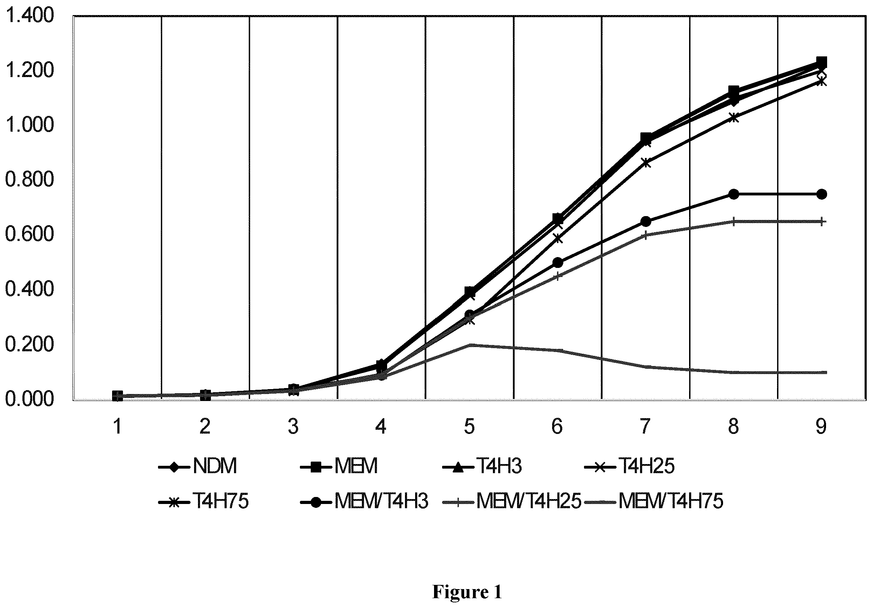

The combined efficacy of MEM and TETA-4HCL was also assessed by performing time-kill assays in Mueller-Hinton broth against the recombinant strain *E. coli* MG1655/pUCP24-NDM-1. MEM was used at concentrations of 8 µg/ml either alone or in combination with different concentrations of TETA-4HCL. Bacterial growth was evaluated by measuring OD600 every hour using a spectrophotometer³¹. The data were expressed as the geometric mean. **Figure 1****,** shows the growth curves of *E. coli* under these conditions, revealing a synergic activity of the combination of MEM and TETA-4-HCL over time that is dose-dependent.

### Example 3: Determination of 50% inhibitory concentration (IC₅₀) of TETA-4HCL

IC₅₀ measurements were performed as previously described²⁴. Briefly, β-lactamases from crude extracts were prepared and used for measuring the specific activity in 100 mM sodium phosphate buffer (pH 7.0). Measurements were performed in a Genesys 10S UV/VIS spectrophotometer using a wavelength of 262 nm for cephalothin. The 50% inhibitory concentrations (IC₅₀) for IMP-1 and NDM-5, taken as examples of MBLs, were determined as the concentrations of TETA-4HCL that reduced the hydrolysis rate of 100 µM cephalothin by 50%. Extracts were preincubated with TETA-4HCL for 3 min prior to the addition of cephalothin. As a comparison, the IC50 of DMSA for IMP-1 and NDM-1 are 0.12 and 1.16 mM, respectively (data not shown). These results also show that the inhibitory effect of TETA-4HCL may depend on the MBL type.

The IC₅₀ value of at least IMP-1 (6900 µM) and NDM-1 (46 µM) indicates the potential of inhibition of TETA-4HCL of the activity of the tested MBL.

**Enzyme IC₅₀ (mM)**

| | |
|---|---|
| IMP-1: | 6.9 |
| IMP-10: | 6.9 |
| NDM-1: | 0.046 |
| NDM-5: | 0.077 |
| NDM-7: | 0.17 |
| NDM-9: | 0.69 |
| VIM-1: | 2.3 |

### Example 4: In vitro test for detecting MBLs and in particular NDM MBLs

This test can be seen as an adapted version of the NitroSpeed-Carba NP test, which has been developed to identify carbapenemase production in Enterobacterales and to discriminate between the different types of clinically significant carbapenemases (Ambler classes A, B, and D)³⁶. It is based on two main features; (i) the hydrolysis of the hydrolysis substrate (nitrocefin), a chromogenic substrate cleaved by all β-lactamases, including carbapenemases, and (ii), the ability of ertapenem (ETP) (a carbapenem) to prevent this hydrolysis for all β-lactamases except carbapenemases.

Specific carbapenemase inhibitors of class A (avibactam, vaborbactam), class B (MBLs) (dipicolinic acid), and class D (avibactam) are also used to inhibit the nitrocefin hydrolysis and to allow the identification of the carbapenemase types with a turnaround time of ca. 30 min.

The test was adapted by using TETA-4HCL (1.3 mg/ml) instead of dipicolinic acid (DPA) for detecting MBL activity.

Generally, five test tubes are prepared, all containing 100 µl of a Tris-HCl 20mmol/liter /lysis buffer (B-PER II bacterial extraction reagent) containing 0.1 mM ZnSO₄.

In tubes 3-5, three different carbapenemase inhibitors are added:
Tube 3: TETA-4HCL, 1.3 mg/ml
Tube 4: AVI (Avibactam), 0.04 mM (12.2 µg/ml)
Tube 5: VAB (Vaborbactam), 50 µg/ml.

One to two calibrated 1 µl-loopfuls of colonies grown overnight are added to each tube, and the tubes are vortexted for 30-60 seconds.

50 µl of ETP solution (80 µg/ml) is added to tubes 2 to 5, and 50 µl of distilled water to Tube 1.

After 5 minutes incubation, the chromogenic substrate, nitrocefin, is added to all tubes.

The read-out is as follows:
In case any β-lactamase is present, Tube 1 will turn colored. Tube 1 thus servers also as a positive control.

In case Tube 2 is colored there is a carabapenemase present, while if it is not colored, the β-lactamase is not a carabapenemase (assuming Tube 1 is colored).

Assuming that Tubes 1 and 2 are colored, a carbapenemase is present. Tubes 3-5 of the test allow determining which class of carbapenemase is present based on substrate hydrolysis in these tubes.

In case Tube 3 is colored, but Tubes 4 and 5 are not, this indicates the presence of a Class A carbapenemase (Klebsiella Pneumoniae Carbapenemase, KPC), since these carbapenemases are inhibited by both AVI and VAB, but not by TETA-4HCL.

In case Tube 3 is colored, Tubes 4 is not but Tube 5 is, this indicates the presence of a Class D carbapenemase, since the latter is inhibited by AVI, while Class D carbapenemases are not inhibited by VAB nor by TETA-4HCL.

If all Tubes 1-5 are colored, this indicates the presence of both, Class B and class D carbapenemases.

If all tubes are colored except Tube 3, this indicates the presence of a class B enzyme, an MBL, which is specifically inhibited by the presence of TETA-4HCL.

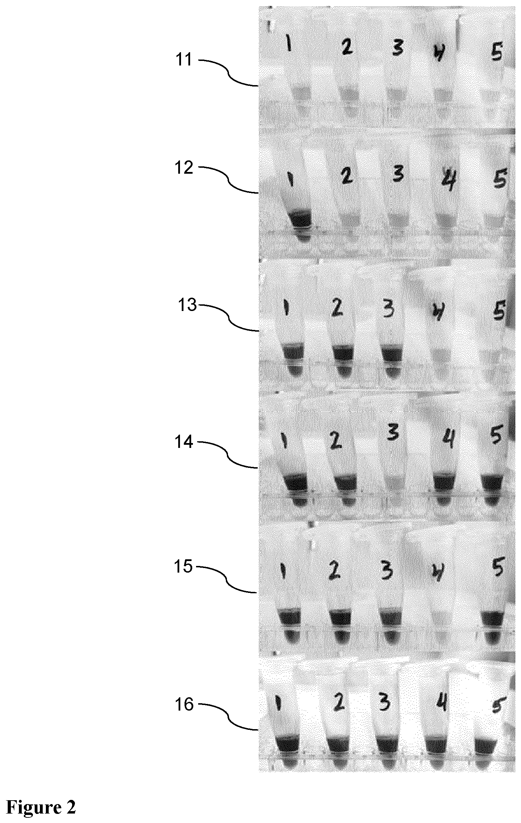

**Figure 2** shows the possible coloring patterns and the interpretation in accordance with the above description. The reference numerals have the following interpretation:
11: No β-lactamase present
12: A non-carbapenemase β-lactamase is present
13: Class A carabepenemase is present
14: Class B carabepenemase is present
15: Class D carabepenemase is present (but no class B carbapenemase)
16: Class B + D carabepenemases are present

### Example 4 thereby also illustrates inhibition of type B carbapenemase by TETA-4HCL in vitro.

### Example 5: In vitro test for distinguishing MBLs from Class A carbapenemase

A test was conducted using three test conditions, using nitrocefin in all cases as color-marker showing carbapenemase activity:
Tube 1, being a control, showing a solution corresponding to Tube 3 above containing NDM-1, but lacking TETA-4HCl,
Tube 2, which contains the TETA-4HCl MBL inhibitor (1.3 mg/nL), and KPC-2 Class A carbapenemase.
Tube 3, corresponding to Tube 3 in Example 4 (NDM-1 carbapenemase, TETA-4HCl inhibitor (1.3 mg/nL).

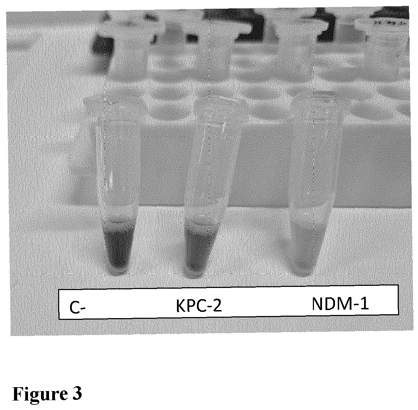

The test was conducted as described above, but ETP was not added, since not required in this setting. The result is shown in **Figure 3****:** Carbapenemase activity is entirely prevented in the case of the MBL carbapenemase (Tube 3), and is not inhibited by TETA 4HCl in Tube 2 (Class A carbapenemase), nor in Tube 1, lacking the TETA 4HCl inhibitor.

While certain of the preferred embodiments of the present invention have been described and specifically exemplified above, it is not intended that the invention be limited to such embodiments. Various modifications may be made thereto without departing from the scope and spirit of the present invention, as set forth in the following claims.

**Table 2: MICS and FICIS of recombinant strains producing different carbapenemases against MEM with or without TETA-4HCL at 500, 250, 125, 75, 25 and 3 mg/L**

| **Part 1: 500 and 250 mg/L (µg/ml)** | | | | | |
|---|---|---|---|---|---|
| *E. coli* | MEM ug/ml | MEM + TETA-4HCL, 500 ug/ml | **FIC** | MEM + TETA-4HCL, 250 ug/ml | **FIC** |
| | | | [Fold change] | | [Fold change] |
| MG1655 | 0.064 | 0.064 | **1.1** [1] | 0.064 | **1.05** [1] |
| MG1655 / PUCP24 | 0.064 | 0.064 | **1.1** [1] | 0.064 | **1.05** [1] |
| MG1655 / OXA-181 | 0.5 | 0.5 | **1.1** [1] | 0.5 | **1.05** [1] |
| MG1655 / KPC-3 | 4 | 4 | **1.1** [1] | 4 | **1.05** [1] |
| MG1655 / VIM-2 | 0.5 | 0.064 | **0.23** [8] | 0.064 | **0.18** [8] |
| MG1655 / IMP-1 | 32 | 1 | **0.13** [32] | 4 | **0.18** [8] |
| MG1655 / IMP-10 | 64 | 0.5 | **0.11** [128] | 2 | **0.08** [32] |
| MG1655 / NDM-1 | 8 | 0.064 | **0.11** [128] | 0,12 | **0.07** [8] |
| MG1655 / NDM-5 | 8 | 0.064 | **0.11** [128] | 0,12 | **0.07** [8] |
| MG1655 / NDM-9 | 32 | 0.12 | **0.1** [256] | 0.12 | **0.05** [256] |

**Table 2 Part 2: 125 and 75 mg/L (µg/ml)**

| *E. coli* | MEM + TETA-4HCL ,125 ug/mlL | **FIC** | MEM + TETA-4HCL ,75 ug/ml | **FIC** |
|---|---|---|---|---|
| | | [Fold change] | | [Fold change] |
| MG1655 | 0.064 | **1.03** [1] | 0.064 | **1.02** [1] |
| MG1655 / PUCP24 | 0.064 | **1.03** [1] | 0.064 | **1.02** [1] |
| MG1655 / OXA-181 | 0.5 | **1.03** [1] | 0.5 | **1.02** [1] |
| MG1655 / KPC-3 | 4 | 1.03 [1] | 4 | **1.02** [1] |
| MG1655 / VIM-2 | 0.064 | **0.15** [8] | 0.064 | **0.14** [8] |
| MG1655 / IMP-1 | 16 | **0.53** [2] | 16 | **0.52** [2] |
| MG1655 / IMP-10 | 8 | **0.15** [8] | 16 | **0.27** [4] |
| MG1655 / NDM-1 | 0.12 | **0.04** [64] | 0.5 | **0.08** [16] |
| MG1655 / NDM-5 | 0.12 | **0.04** [8] | 0.25 | **0.05** [32] |
| MG1655 / NDM-9 | 0.5 | **0.04** [64] | 1 | **0.05** [32] |

**Table 2 Part 3: 25 and 3 µg/ml (µg/ml),**

| *E. coli* | MEM + TETA-4HCL, 25 ug/ml | **FIC** | MEM + TETA-4HCL, 3 ug/ml | **FIC** |
|---|---|---|---|---|
| | | [Fold change] | | [Fold change] |
| MG1655 | 0.064 | **1.01** [1] | 0.064 | **1.01** [1] |
| MG1655 / PUCP24 | 0.064 | **1.01** [1] | 0.064 | **1.01** [1] |
| MG1655 / OXA-181 | 0.5 | **1.01** [1] | 0.5 | **1.01** [1] |
| MG1655 / KPC-3 | 4 | **1.01** [1] | 4 | **1.01** [1] |
| MG1655 / VIM-2 | 0.12 | **0.26** [4] | 0.5 | **1.01** [1] |
| MG1655 / IMP-1 | 32 | **1.01** [1] | 32 | **1.01** [1] |
| MG1655 / IMP-10 | 32 | **0.51** [2] | 64 | **1.01** [1] |
| MG1655 / NDM-1 | 2 | **0.26** [4] | 4 | **0.51** [2] |
| MG1655 / NDM-5 | 1 | **0.13** [8] | 2 | **0.26** [4] |
| MG1655 / NDM-9 | 8 | **0.26** [4] | 8 | **0.3** [4] |

### References

1. Nordmann P, Poirel L. Epidemiology and Diagnostics of Carbapenem Resistance in Gram-negative Bacteria. Clinical Infectious Diseases 2019; 69: S521-8. https://doi.org/10.1093/cid/ciz824
2. van Duin D, Kaye KS, Neuner EA, Bonomo RA. Carbapenem-resistant Enterobacteriaceae: a review of treatment and outcomes. Diagnostic microbiology and infectious disease 2013; 75: 115-20. https://doi.org/10.1016/j.diagmicrobio.2012.11.009
3. Cai B, Echols R, Magee G, et al. Prevalence of Carbapenem-Resistant Gram-Negative Infections in the United States Predominated by Acinetobacter baumannii and Pseudomonas aeruginosa. Open Forum Infectious Diseases 2017; 4: ofx176. https://doi.org/10.1093/ofid/ofx176
4. World Health Organization (WHO). Global priority list of antibiotic-resistant bacteria to guide research, discovery, and development of new antibiotics. 2017. Available at: http://remed.org/wp-content/uploads/2017/03/lobal-priority-list-of-antibiotic-resistant-bacteria-2017.pdf. Accessed March 15, 2024.
5. Murray CJL, Ikuta KS, Sharara F, et al. Global burden of bacterial antimicrobial resistance in 2019: a systematic analysis. The Lancet 2022; 399: 629-55. https://doi.org/10.1016/S0140-6736(21)02724-0
6. Nordmann P, Poirel L. Strategies for identification of carbapenemase-producing Enterobacteriaceae. Journal of Antimicrobial Chemotherapy 2013; 68: 487-9. https://doi.org/10.1093/jac/dks426
7. Ambler RP. The Structure of beta-Lactamases. Philosophical Transactions of the Royal Society B: Biological Sciences 1980; 289: 321-31. http://rstb.royalsocietypublishing.org/cgi/doi/10.1098/rstb.1980.0049
8. Bush K, Jacoby GA. Updated Functional Classification of β-Lactamases. Antimicrobial agents and chemotherapy 2010; 54: 969-76. https://doi.org/10.1128/AAC.01009-09
9. Bush K, Bradford PA. Epidemiology of β-Lactamase-Producing Pathogens. Clinical microbiology reviews 2020; 33: e00047-19. https://doi.org/10.1128/CMR.00047-19
10. Cuzon G, Naas T, Truong H, et al. Worldwide Diversity of Klebsiella pneumoniae That Produce β-Lactamase bla KPC-2 Gene1. Emerging infectious diseases 2010; 16: 1349-56. http://wwwnc.cdc.gov/eid/article/16/9/09-1389_article.htm.
11. Smith Moland E. Plasmid-mediated, carbapenem-hydrolysing beta-lactamase, KPC-2, in Klebsiella pneumoniae isolates. Journal of Antimicrobial Chemotherapy 2003; 51: 711-4. https://doi.org/10.1093/jac/dkg124
12. Queenan AM, Bush K. Carbapenemases: the Versatile β-Lactamases. Clinical microbiology reviews 2007; 20: 440-58. Available at: https://cmr.asm.org/content/20/3/440.
13. Poirel L, Héritier C, Tolün V, Nordmann P. Emergence of Oxacillinase-Mediated Resistance to Imipenem in Klebsiella pneumoniae. Antimicrobial agents and chemotherapy 2004; 48: 15-22. https://doi.org/10.1128/AAC.48.1.15-22.2004
14. Pitout JDD, Peirano G, Kock MM, Strydom K-A, Matsumura Y. The Global Ascendency of OXA-48-Type Carbapenemases. Clinical microbiology reviews 2019; 33: e00102-19. https://doi.org/10.1128/CMR.00102-19
15. Mairi A, Pantel A, Sotto A, Lavigne J-P, Touati A. OXA-48-like carbapenemases producing Enterobacteriaceae in different niches. European Journal of Clinical Microbiology & Infectious Diseases 2018; 37: 587-604. https://doi.org/10.1007/s10096-017-3112-7
16. López C, Delmonti J, Bonomo RA, Vila AJ. Deciphering the evolution of metallo-β-lactamases: A journey from the test tube to the bacterial periplasm. Journal of Biological Chemistry 2022; 298: 101665. https://doi.org/10.1016/j.jbc.2022.101665
17. Bush K. The ABCD's of β-lactamase nomenclature. Journal of Infection and Chemotherapy 2013; 19: 549-59. https://doi.org/10.1007/s10156-013-0640-7
18. Segatore B, Massidda O, Satta G, Setacci D, Amicosante G. High specificity of cphA-encoded metallo-beta-lactamase from Aeromonas hydrophila AE036 for carbapenems and its contribution to beta-lactam resistance. Antimicrobial agents and chemotherapy 1993; 37: 1324-8. https://doi.org/10.1128/AAC.37.6.1324
19. González LJ, Vila AJ. Carbapenem Resistance in Elizabethkingia meningoseptica Is Mediated by Metallo-β-Lactamase BlaB. Antimicrobial agents and chemotherapy 2012; 56: 1686-92. https://doi.org/10.1128/AAC.05835-11
20. Akova M, Bonfiglio G, Livermore DM. Susceptibility to β-lactam antibiotics of mutant strains of Xanthomonas maltophilia with high- and low-level constitutive expression of L1 and L2 β-lactamases. Journal of medical microbiology 1991; 35: 208-13. https://doi.org/10.1099/00222615-35-4-208
21. Boyd SE, Livermore DM, Hooper DC, Hope WW. Metallo-β-Lactamases: Structure, Function, Epidemiology, Treatment Options, and the Development Pipeline. Antimicrobial agents and chemotherapy 2020; 64 : e00397-20. https://doi.org/10.1128/AAC.00397-20
22. Wachino J, Yoshida H, Yamane K, et al. SMB-1, a Novel Subclass B3 Metallo-β-Lactamase, Associated with IS CR1 and a Class 1 Integron, from a Carbapenem-Resistant Serratia marcescens Clinical Isolate. Antimicrobial agents and chemotherapy2011; 55: 5143-9. https://doi.org/10.1128/AAC.05045-11
23. Zhou H, Guo W, Zhang J, Li Y, Zheng P, Zhang H. Draft genome sequence of a metallo-β-lactamase (blaAIM-1)-producing Klebsiella pneumoniae ST1916 isolated from a patient with chronic diarrhoea. Journal of global antimicrobial resistance 2019; 16: 165-7. https://doi.org/10.1016/j.jgar.2021.06.010
24. Watanabe M, Iyobe S, Inoue M, Mitsuhashi S. Transferable imipenem resistance in Pseudomonas aeruginosa. Antimicrobial agents and chemotherapy 1991; 35: 147-51. https://doi.org/10.1128/AAC.35.1.147
25. Lauretti L, Riccio ML, Mazzariol A, et al. Cloning and Characterization of blaVIM , a New Integron-Borne Metallo-β-Lactamase Gene from a Pseudomonas aeruginosa Clinical Isolate. Antimicrobial agents and chemotherapy 1999; 43: 1584-90. https://doi.org/10.1128/AAC.43.7.1584
26. Poirel L, Naas T, Nicolas D, et al. Characterization of VIM-2, a Carbapenem-Hydrolyzing Metallo-β-Lactamase and Its Plasmid- and Integron-Borne Gene from a Pseudomonas aeruginosa Clinical Isolate in France. Antimicrobial agents and chemotherapy 2000; 44: 891-7. htts://doi.org/10.1128/AAC.44.4.891-897.2000
27. Yong D, Toleman MA, Giske CG, et al. Characterization of a New Metallo- beta-Lactamase Gene, blaNDM-1, and a Novel Erythromycin Esterase Gene Carried on a Unique Genetic Structure in Klebsiella pneumoniae Sequence Type 14 from India. Antimicrobial agents and chemotherapy 2009; 53: 5046-54. https://doi.org/10.1128/AAC.00774-09
28. Gales AC. Dissemination in distinct Brazilian regions of an epidemic carbapenem-resistant Pseudomonas aeruginosa producing SPM metallo-β-lactamase. Journal of Antimicrobial Chemotherapy 2003; 52: 699-702. https://doi.org/10.1093/jac/dkg416
29. Yang Y, Yan Y-H, Schofield CJ, McNally A, Zong Z, Li G-B. Metallo-β-lactamase-mediated antimicrobial resistance and progress in inhibitor discovery. Trends in microbiology 2023; 31: 735-48. https://doi.org/10.1016/j.tim.2023.01.013
30. Adler A, Ghosh H, Gross A, et al. Clinical and molecular features of NDM-producing Acinetobacter baumannii in a multicenter study in Israel. Annals of clinical microbiology and antimicrobials 2023; 22: 52. https://doi.org/10.1186/s12941-023-00607-w
31. Zavascki AP., Barth AL, Gongalves, ALS, et al. The influence of metallo-β-lactamase production on mortality in nosocomial Pseudomonas aeruginosa infections. Journal of Antimicrobial Chemotherapy 2006; 58: 387-92. https://doi.org/10.1093/jac/dkl239
32. Persoon MC, Voor in 't holt AF, van Meer MPA, et al. Mortality related to Verona Integron-encoded Metallo-β-lactamase-positive Pseudomonas aeruginosa: assessment by a novel clinical tool. Antimicrobial resistance and infection control 2019; 8: 107. https://doi.org/10.1186/s13756-019-0556-9
33. Soneda K, Uda K, Araki K, et al. Clinical characteristics and treatment of IMP-type carbapenemase-producing Enterobacteriaceae bacteremia: Case series and literature review. Journal of Infection and Chemotherapy 2023; 29: 26-32. https://doi.org/10.1016/j.jiac.2022.09.003
34. Snyder BM, Montague BT, Anandan S, et al. Risk factors and epidemiologic predictors of blood stream infections with New Delhi Metallo-b-lactamase (NDM-1) producing Enterobacteriaceae. Epidemiology and infection 2019; 147: e137. https://doi.org/10.1017/S0950268819000256
35. Tooke CL, Hinchliffe P, Bragginton EC, et al. β-Lactamases and β-Lactamase Inhibitors in the 21st Century. Journal of molecular biology 2019; 431: 3472-500. https://doi.org/10.1016/j.jmb.2019.04.002
36. Campanella TA, Gallagher JC. Clinical Review and Critical Evaluation of Imipenem-Relebactam: Evidence to Date. Infection and drug resistance 2020; Volume 13: 4297-308. https://doi.org/10.2147/IDR.S224228
37. Abboud MI, Damblon C, Brem J, et al. Interaction of Avibactam with Class B Metallo-β-Lactamases. Antimicrobial agents and chemotherapy 2016; 60: 5655-62. https://doi.org/10.1128/AAC.00897-16
38. Lomovskaya O, Sun D, Rubio-Aparicio D, et al. Vaborbactam: Spectrum of beta-lactamase inhibition and impact of resistance mechanisms on activity in Enterobacteriaceae. Antimicrobial agents and chemotherapy 2017; 61: e01443-17. https://doi.org/10.1128/AAC.01443-17
39. Biedenbach DJ, Kazmierczak K, Bouchillon SK, Sahm DF, Bradford PA. In Vitro Activity of Aztreonam-Avibactam against a Global Collection of Gram-Negative Pathogens from 2012 and 2013. Antimicrobial agents and chemotherapy 2015; 59: 4239-48. https://doi.org/10.1128/AAC.00206-15
40. Sadek M, Juhas M, Poirel L, Nordmann P. Genetic Features Leading to Reduced Susceptibility to Aztreonam-Avibactam among Metallo-β-Lactamase-Producing Escherichia coli Isolates. Antimicrobial agents and chemotherapy2020; 64: e01659-20. https://doi.org/10.1128/AAC.01659-20
41. Mauri C, Maraolo AE, Di Bella S, Luzzaro F, Principe L. The Revival of Aztreonam in Combination with Avibactam against Metallo-β-Lactamase-Producing Gram-Negatives: A Systematic Review of In Vitro Studies and Clinical Cases. Antibiotics 2021; 10: 1012. https://doi.org/10.3390/antibiotics10081012
42. Yahav D, Giske CG, Gra̅matniece A, Abodakpi H, Tam VH, Leibovici L. New β-Lactam-β-Lactamase Inhibitor Combinations. *Clinical microbiology reviews* 2020; **34:** e00115-20. https://doi.org/10.1128/CMR.00115-20
43. Lomovskaya O, Castanheira M, Lindley J, et al. In vitro potency of xeruborbactam in combination with multiple β-lactam antibiotics in comparison with other β-lactam/β-lactamase inhibitor (BLI) combinations against carbapenem-resistant and extended-spectrum β-lactamase-producing Enterobacterales. Antimicrobial agents and chemotherapy 2023; 67 : e0044023. https://doi.org/10.1128/aac.00440-23
44. Le Terrier C, Nordmann P, Buchs C, et al. Wide dissemination of Gram-negative bacteria producing the taniborbactam-resistant NDM-9 variant: a One Health concern. Journal of Antimicrobial Chemotherapy 2023; 78: 2382-4. https://doi.org/10.1093/jac/dkad210
45. Cheminet G, de Lastours V, Poirel L, et al. Dimercaptosuccinic acid in combination with carbapenems against isogenic strains of Escherichia coli producing or not producing a metallo-β-lactamase in vitro and in murine peritonitis. Journal of Antimicrobial Chemotherapy 2020; 75: 3593-600. https://doi.org/10.1093/jac/dkaa347
46. Bouvier M, Freire S, Findlay J et al. In-vitro activity of dimercaptosuccinic acid in combination with carbapenems agasint carbapenems-resistant Pseudomonas aeruginosa. Microbial Drug Resistance. 2025 31;16:20. https://doi: 10.3390/antibiotics10050558
47.Nordmann P, Herrera-Espejo S, Bouvier M, Poirel L, Pachon Ibanez ME. Dimercaptosuccinic acid in combination with carbapenems against strains of Pseudomonas aeruginosa and Acinetobacter baumannii producing metallo-β-lactamase in a murine peritonitis model. In: 8th AMR Conference, 6-7 March 2024. Basel.
48. Principe L, Vecchio L, Sheehan G et al. Zinc chelators as carbapenem adjuvants for metallo-β-lactamase-producing bacteria; in vitro and in vivo evaluation. Microbial Drug Resistance 2020; 26:1133-1143. https:// doi: 10.1089/mdr.2020.0037.
49. Gonzalez-Bello, Rodriguez D, Pernas M. et al. β-lactamase inhibitors to restor the efficacy of antibiotics against superbugs. Journal of Medical Chemistry, 2020 63:1859-1881, https//doi:10.1021/acs.jmedchem9b01279.
50. Sychantha D, Rotondo C, Tehrani KHME et al. Aspergillomarasmine A inhibits metallo-β-lactamases by selectively sequestring Zn. Journal of Biological Chemistry 2021; 297 https://doi.org/10.1016/j/jbc.2021.100918.
51. Pfeiffenberger J, Kruse C, Mutch P, Harker A, Weiss KH. The steady state pharmacokinetics of trientine in Wilson disease patients. Eur J Clin Pharmacol. 2018 Jun;74(6):731-736. doi: 10.1007/s00228-018-2424-6. Epub 2018 Feb 7. PMID: 29417175.

## Claims

1. A compound comprising a moiety of formula (I) below, protonated and deprotonated forms thereof, and/or of a pharmaceutically acceptable salt thereof, for treating a bacterial infection and/or a disease caused by bacteria 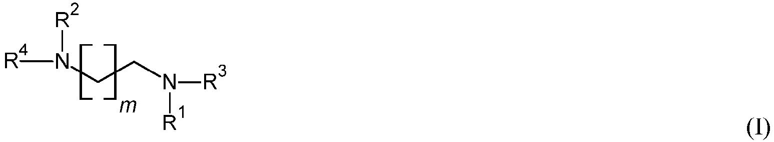 wherein
m is 0 and integers of 1-5, preferably 1;
R¹, R², R³, and R⁴ are independently selected from H and substituents of formulae (1) below 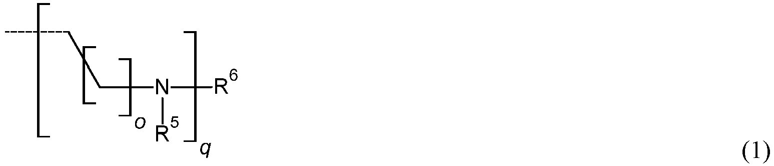
wherein
o is selected from 0 and integers of 1-5, preferably 1;
q is an integer of 1-5;
R⁵ and R⁶ are independently selected from H and substituents of formula (2) below 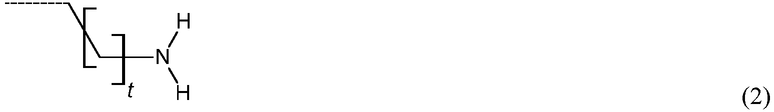
wherein
t is selected from 0 and integers of 1-5.

2. The compound of claim 1, wherein R¹, R², R³, and R⁴ are independently selected from H and substituents of formulae (3), (4), and (5) below

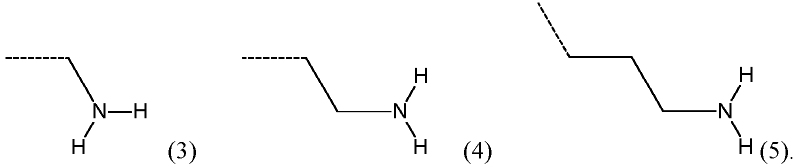

3. The compound of claim 1 or claim 2, wherein m is 1.

4. The compound of any one of claims 1-3, wherein R¹ and R² are H, and R³, and R⁴ are independently selected from substituents of formulae (3), (4), and (5).

5. The compound of any one of claims 1-4, wherein the moiety of formula (I) is or comprises triethylenetetramine (TETA).

6. The compound of any one of the preceding claims, which is provided in the form of an amine salt.

7. The compound of any one of the preceding claims, which is provided in the form of a hydrochloride salt, preferably a tretrahydrochloride (4HCl) or dihydrochloride (2HCl) salt.

8. The compound of any one of the preceding claims, for use the treatment of infections of carabapenemase producing bacteria and/or carbapenem-resistant bacteria.

9. The compound of any one of the preceding claims, for use the treatment of infections of metallo-β-lactamase (MBL) producing bacteria.

10. The compound of any one of the preceding claims, for use in the treatment of infections of bacteria producing an MBL subclass B1, B2 and/or B3 carbapenemase.

11. The compound of any one of the preceding claims, for use in the treatment of infections of bacteria producing one or more selected from the group consisting of: New Delhi Metallo-β-lactamase (NDM), imipenemase (IMP) and Verona imipenemase (VIM).

12. The compound for use of any one of the preceding claims, in combination with a β-lactam antibiotic, preferably a carbapenem antibiotic, for example selected from meropenem and imipenem.

13. The compound of any one of the preceding claims, which is administered at a dose of 150 mg to 10 g per day, preferably 200 mg to 6 g per day, even more preferably 250 mg to 5 g per day, and most preferably 300 mg to 3,5 g per day.

14. An *in vitro* and/or *ex vivo* test kit for detecting the presence of a carbapenemase, preferably a metallo-β-lactamase (MBL), the kit comprising the compound of formula (I).

15. An *in vitro* method for detecting the presence of a metallo-β-lactamase (MBL), the method comprising:
- providing a sample comprising bacterium to be tested and the compound of formula (I);
- detecting the presence of said MBL if an activity of said MBL is inhibited due to the presence of said compound of formula (I).

16. The method of claim 15, wherein said sample further comprises a substrate of said MBL, and wherein said activity is determined by detecting the disappearance of the substrate and/or the occurrence of a product that is the result of said activity.

17. A method for treating a bacterial infection, the method comprising administering, to a subject in need thereof, a therapeutically effective amount of the compound of formula (I). [

18. The method of claim 17, wherein a bacterium causing said bacterial infection is a carbapenem producing bacterium, preferably an MBL producer, and most preferably a B1 subclass MBL producer.

19. The method of any one of claims 17 and 18, which comprises administering a carbapenem antibiotic.
